# EUROPEAN PATENT APPLICATION

(11) **EP 4 621 503 A1**
(43) Date of publication of application: **24.09.2025**
(21) Application number: 24164237.0
(22) Date of filing: 18.03.2024
(51) Int. Cl.: G05B 13/02, G05B 19/418, B04B 15/06, G05B 23/02, B08B 9/02, B08B 9/08

(54) **METHOD AND SYSTEM FOR PREDICTING THE QUALITY OF A CLEANING-IN-PLACE CYCLE OF AN INDUSTRIAL PRODUCTION LINE**

(71) Applicant: GEA Westfalia Separator Group GmbH, 59302 Oelde (DE)
(72) Inventor: WITTKAEMPER, Lars, 48149 Münster (DE); VEER, Thomas, 49832 Beesten (DE); BUSSMANN, Daniel, 48739 Legden (DE); IRRGANG, Sven, 59302 Oelde (DE)
(74) Representative: Bardehle Pagenberg Partnerschaft mbB Patentanwälte Rechtsanwälte

(57) **Abstract**

The present disclosure relates to a computer-implemented method for predicting the quality of a Cleaning-in-Place (CIP) cycle of an industrial production line comprising at least one equipment and at least one CIP station, the method comprising the steps of: executing, by the CIP station, the CIP cycle; identifying one or more CIP steps of the CIP cycle; selecting, for each of the identified one or more CIP steps, a trained process-specific machine learning model associated with the CIP station according to the identified one or more CIP steps; passing one or more CIP station-specific parameters to the trained process-specific machine learning model; and predicting the quality of the CIP cycle based on the one or more CIP station-specific parameters, wherein the quality is reflected by an output of each of the trained process-specific machine learning models. In addition, the present disclosure relates to a corresponding control computing system, to an industrial production line comprising or communicatively coupled to at least one control computing system, to computer-implemented methods for training a process-specific and an equipment-specific machine learning model, a trained machine learning model as well as to corresponding computer programs.

## Description

### Technical field of the invention

The present disclosure concerns a computer-implemented method for predicting the quality of a Cleaning-in-Place cycle of an industrial production line comprising at least one equipment and at least one Cleaning-in-Place station as well as to a corresponding control computing system and a corresponding industrial production line. In addition, the present disclosure concerns computer-implemented methods for training corresponding machine learning models. Furthermore, the present disclosure concerns computer programs for carrying out the aforementioned methods.

### Technical background

Specifically in the food and beverage industry, like the dairy industry, the maintenance of stringent hygiene standards is paramount to uphold product quality and comply with regulatory requirements. Cleaning-in-Place (CIP) stations, typically attached to industrial production lines in the realm of the food and beverage industry, have emerged as indispensable components in industrial production lines, dedicated to ensuring the cleanliness and sanitization of various equipment or components critical to the production process.

Typically, a CIP solution (comprising caustic, acid and/or water) is circulated by a CIP station through pipelines, tanks, and equipment of the industrial production line using pumps in different steps of a plurality of steps of a CIP cycle, wherein a CIP cycle may comprise CIP steps like pre-flushing, sanitizing, flushing etc. This circulation process dislodges and removes product residues, contaminants, and microbial growth from the industrial production line and its equipment. Temperature control may be incorporated to optimize the cleaning process, with elevated temperatures enhancing the effectiveness of cleaning agents. The CIP solution circulates for a predetermined duration to ensure thorough cleaning and sanitation. Afterwards, a rinsing phase is initiated to remove the CIP solution and residues from the industrial production line and its equipment.

A persistent challenge plaguing known CIP stations lies in the absence of robust mechanisms for evaluating the quality of the cleaning cycles, also referred to as CIP cycles, applied. Specifically, existing systems lack overall and equipment-specific monitoring, validation, or plausibility checks that are essential for verifying the cleaning effectiveness of industrial production lines and more particularly of specific industrial equipment, such as separators or decanters, used within industrial production lines.

Monitoring and quality control of a cleaning process of industrial production lines are nowadays typically done by measuring relevant parameters directly in the return flow of the CIP solutions at the CIP station, which may provide an overall indication on the quality and effectiveness of the CIP cycles. However, this does not provide any detailed information in relation to the quality of cleaning of the industrial production line and of the specific equipment in the industrial production line. Furthermore, no indication is provided with respect to the execution of individual steps of a CIP cycle. Moreover, securing a proper cleaning quality, specifically when operating a special equipment like a separator or decanter, requires analysis of additional measurements and process values, which cannot be detected in the return flow of the CIP solutions at the CIP station as they are locally measured in the industrial production line or in the specific equipment. In addition, equipment-specific operating parameters and process conditions are typically only set once during the commissioning of the industrial production line or of the specific equipment by a dedicated commissioning engineer following some standard rules. These parameters are typically never changed throughout the lifetime of the industrial production line or of the specific equipment. In addition, after commissioning, the plant operator has no specific knowledge how the system is performing or if adjustments are needed to secure a sufficient cleaning quality.

An insufficient cleaning can at best lead to a faulty operation of the industrial production line or of the specific equipment, causing an efficiency drop due to product losses and/or problems in downstream processes, or in the worst case to a complete production stop due to major issues with the industrial production line.

Recognizing these issues, the need for an improved approach to evaluate the quality of CIP cycles applied to industrial production lines and/or applied to specific equipment of industrial production lines is apparent.

### Summary of the invention

The above-mentioned objects and other objects, which become apparent from the following description, are solved at least in part by the subject-matter of the independent claims. Preferred embodiments are subject of the dependent claims.

According to a 1^{st} aspect of the invention, a computer-implemented method for predicting the quality of a Cleaning-in-Place (CIP) cycle of an industrial production line comprising at least one equipment and at least one CIP station is provided, the method comprising the steps of: executing, by the CIP station, the CIP cycle; identifying one or more CIP steps of the CIP cycle; selecting, for each of the identified one or more CIP steps, a trained process-specific machine learning model associated with the CIP station according to the identified one or more CIP steps; passing one or more CIP station-specific parameters to the trained process-specific machine learning model; and predicting the quality of the CIP cycle based on the one or more CIP station-specific parameters, wherein the quality is reflected by an output of each of the trained process-specific machine learning models.

Using an individually trained process-specific machine learning model per CIP step rather than one model for the complete CIP cycle to evaluate the quality of the corresponding CIP cycle may lead to an improved CIP cycle quality evaluation. This is because the corresponding trained process-specific machine learning model may be specialized on providing only predictions for the corresponding CIP step. This allows, for example, using a specifically trained model for a flushing step and another specifically trained model for a sanitizing step, resulting in improved prediction results compared to using only one model trained for predicting a quality of the overall CIP cycle. It is to be noted that the term CIP step may also refer to parts of a CIP cycle that comprises more than one step.

Using one or more CIP station-specific parameters as input for the trained process-specific machine learning model may provide meaningful prediction results and the parameters can all be obtained directly from the CIP station. Hence, no parameters from other equipment must be collected, resulting in a simplified overall setup.

In addition, using a trained process-specific machine learning model per CIP step simplifies the integration of further models for further CIP steps that might be added to a CIP cycle over the course of time, resulting in an improved scalability. If only one overall model for the complete CIP cycle would be used, this model would have to be retrained or replaced by another model in case that a further CIP step is added to the CIP cycle. If one model per CIP step is used, a further model could be trained for this new step and could be integrated into the overall process without affecting the operation of the models used for the other CIP steps. If more than one trained process-specific machine learning model is used, which may be the case if more than one CIP step has been identified, the outputs of the models may be aggregated to indicate the quality of the CIP cycle.

According to a 2^{nd} aspect in the 1^{st} aspect, the method further comprises the steps of selecting, for each of the identified one or more CIP steps, a trained equipment-specific machine learning model associated with the at least one equipment of the production line, passing one or more equipment-specific parameters to the trained equipment-specific machine learning model, and wherein the predicting the quality of the CIP cycle is further based on the one or more equipment-specific parameters, wherein the quality is reflected by an output of each of the trained equipment-specific machine learning models.

Using a trained equipment-specific machine learning model per CIP step in addition to the one or more trained process-specific machine learning models may further improve the prediction of the quality of the CIP cycle since the trained equipment-specific machine learning model is specialized in interpreting parameters obtained from equipment used in in an industrial production line, like separators, decanters, tanks etc.

While the trained process-specific machine learning model takes into account parameters of the CIP station itself, the trained equipment-specific machine learning model takes into account parameters obtained from specific equipment. Hence, both the behavior of the CIP station and the behavior of the equipment installed within the industrial production line is considered. If more than one trained equipment-specific machine learning model is used, which may be the case if more than one CIP step has been identified, the outputs of the models may be aggregated to indicate the quality of the CIP cycle.

According to a 3^{rd} aspect in the 2^{nd} aspect, the predicting the quality of the CIP cycle further comprises aggregating, when the CIP cycle is completed, the outputs of each of the trained process-specific machine learning models and of each of the trained equipment-specific machine learning models to generate a quality indicator.

Aggregating the outputs of the trained machine learning models may provide one result that expresses the quality of the CIP cycle with respect to the whole industrial production line, including specific equipment used therein. Having only one aggregated value in terms of a quality indicator may allow a simplified interpretation, e.g., by service personnel.

According to a 4^{th} aspect in the 2^{nd} and/or 3^{rd} aspect, the at least one equipment is a separation device, preferably a decanter or a separator, and the one or more equipment-specific parameters comprise one or more of: a bowl speed of the at least one equipment, a motor current of the at least one equipment, an ejection indicator indicating whether a total ejection or a partial ejection has been conducted by the at least one equipment, a vibration rate of the at least one equipment, a flow rate to the equipment, and/or a discharge pressure or backpressure rate.

Since decanters and separators are complex devices that require specific CIP steps for being cleaned, the usage of a specifically trained machine learning model for these equipment types is beneficial as such models may be tailored to work meaningful for such device types.

The bowl speed, the motor current, the injection indicator, the vibration rate, the flow rate and the discharge pressure rate (also referred to as backpressure rate) are all in direct correlation to the cleaning quality of a specific equipment, like a separator or a decanter. The interaction of these parameters in particular allows direct conclusions to be drawn about the quality of the cleaning process. If, for example, the discharge pressure rate is lower or higher as usual, this may indicate that problems occurred during the corresponding CIP step, possibly resulting in an insufficiently thorough cleaning process with respect to a specific equipment.

According to a 5^{th} aspect in one of the preceding aspects, the one or more CIP station-specific parameters comprise one or more of: a state of the industrial production line, a flow rate, a backflow temperature, a line temperature, and/or a conductivity.

The state of the industrial production line, the flow rate, the backflow temperature, the line temperature, and the conductivity are parameters that can be directly obtained from a common CIP station. Thus, no refitting of the CIP station is required, i.e., no additional sensors need to be added to common CIP stations, which simplifies the usage of the method in conjunction with common CIP stations.

In addition, the aforementioned parameters are all in direct correlation to the cleaning quality of an industrial production line. The interaction of these parameters in particular allows direct conclusions to be drawn about the quality of the cleaning process. If, for example, the flow rate is lower or higher as usual, this may indicate that problems occurred during the corresponding CIP step, possibly resulting in an insufficiently thorough cleaning process with respect to the industrial production line.

According to a 6^{th} aspect in the 5^{th} aspect, the step of identifying one or more CIP steps of the CIP cycle is based on at least one of: the backflow temperature and/or the conductivity, and/or data provided by the CIP station and/or by the production line.

Since the backflow temperature, the conductivity, and data provided by the CIP station deviate between different CIP steps, all of these parameters are well suited for identifying the CIP step that is presently executed.

According to a 7^{th} aspect in one of the preceding aspects, the trained process-specific machine learning model is one of: a Support Vector Machine, preferably an Optimized Support Vector Machine, a Random Forest, preferably an Optimized Random Forest, a Nearest Neighbors algorithm, preferably an Optimized Nearest Neighbors algorithm, an Autoencoder, or one or more Self Organizing Maps (SOMs).

According to an 8^{th} aspect in one of the 2^{nd} to 4^{th} aspects, the trained equipment-specific machine learning model is one of: a Support Vector Machine, preferably an Optimized Support Vector Machine, a Random Forest, preferably an Optimized Random Forest, a Nearest Neighbors algorithm, preferably an Optimized Nearest Neighbors algorithm, an Autoencoder, or one or more SOMs.

Each of the aforementioned model types is usable for performing the prediction of the quality of a CIP cycle. Supporting different types of models allows leveraging the individual benefits provided by the model.

Support Vector Machines (SVM) are effective in high-dimensional spaces and offer versatility through the specification of various kernels. However, they may not be suitable for large datasets due to long training times and sensitivity to kernel parameters. Additionally, SVMs do not provide direct probability estimates.

Random Forests are robust to overfitting, thanks to the averaging of multiple trees, and perform well with high-dimensional data. However, they can be slow to evaluate on large datasets and lack interpretability compared to simpler models. Furthermore, as the number of trees increases, so does the demand for memory and computational resources.

The Nearest Neighbors algorithm is simple to understand and implement, with no training phase required, making it efficient for lazy learning. However, it becomes computationally expensive during prediction, especially with large datasets, and consumes high memory as it needs to store all training data. It's also sensitive to the choice of distance metric.

Autoencoders offer unsupervised learning capability, making them useful for dimensionality reduction and feature learning. However, they require careful tuning of hyperparameters and are susceptible to overfitting, particularly with small datasets. Additionally, their interpretability can be challenging.

Self Organizing Maps (SOM) are useful for visualizing and understanding high-dimensional data, as they can detect and represent non-linear relationships effectively. However, training can be slow, especially with large datasets, and the interpretability of resulting maps may be challenging. Moreover, SOMs require careful initialization and parameter tuning.

According to a 9^{th} aspect, a control computing system for predicting the quality of a CIP cycle of an industrial production line comprising at least one equipment is provided, the control computing system comprising means for performing the method of any one of the 1^{st} to 8^{th} aspect.

According to a 10^{th} aspect, an industrial production line comprising or communicatively coupled to at least one control computing system according to the 9^{th} aspect is provided.

An industrial production line that comprises the control computing system according to the 10^{th} aspect may have the advantage of providing security since sensitive parameters are processed locally. In addition, a further advantage of an industrial production line comprising the control computing system may be low latency due to a reduced transfer time of the parameters to the control computing system. Moreover, independence from network connectivity might be a further advantage. It is to be noted that the industrial production line may comprise a CIP station.

An industrial production line that is communicatively coupled to the control computing system may have the advantage of executing any one of the 1^{st} to 8^{th} aspect in a distributed manner. For instance, the parameters can be obtained at the industrial production line and send to a cloud server where the trained machine learning models that perform the prediction are executed. This provides flexibility with regards to the architectural landscape of information technology. In addition, a distributed environment may provide the advantage of being easily scalable, for example in cases where the demand for computing power increases. In addition, the same control computing system may be used to monitor a plurality of industrial production lines.

According to an 11^{th} aspect, a computer-implemented method for training a process-specific machine learning model adapted to predict the quality of a CIP cycle of an industrial production line is provided, the method comprising obtaining training data from a plurality of parameter sets comprising at least one of: a state of the industrial production line, a flow rate, a discharge pressure rate, a backflow temperature, and/or a conductivity. The method further comprises training the process-specific machine learning model based on the training data to generate a trained process-specific machine learning model.

The above-mentioned advantages apply to this aspect. Moreover, training the process-specific machine learning model based on training data to generate a trained process-specific machine learning model may have the advantage of achieving a model that is tailored to accurately predict the quality of CIP cycles based on CIP station-specific parameters.

According to a 12^{th} aspect, a computer-implemented method for training an equipment-specific machine learning model adapted to predict the quality of a CIP cycle of an industrial production line is provided, wherein the equipment-specific machine learning model is associated with a separation device, preferably a decanter or a separator, the method comprising obtaining training data from a plurality of parameter sets comprising at least one of: a bowl speed of the separation device, a motor current of the separation device, an ejection indicator indicating whether a total ejection or a partial ejection has been conducted by the separation device, and/or a vibration rate of the separation device. The method further comprises training the equipment-specific machine learning model based on the training data to generate a trained equipment-specific machine learning model.

The above-mentioned advantages apply to this embodiment. Moreover, training the equipment-specific machine learning model based on training data to generate a trained equipment-specific machine learning model may have the advantage of achieving a model that is tailored to accurately predict the quality of CIP cycles based on equipment-specific parameters.

According to a 13^{th} aspect in the 11^{th} or 12^{th} aspect, the method further comprises obtaining further training data from an industrial production line during operation, retraining the trained process-specific machine learning model or the trained equipment-specific machine learning model based on the further training data.

Retraining the trained machine learning model models based on the further training data may have the advantage of increasing prediction accuracy over time during operation. In this way, the models may also be adapted during operation to different configurations of the industrial production line and/or the specific equipment that may change over time.

A further advantage of retraining the trained machine learning model is that this technique allows the construction of models that are specialized in predicting the quality of a CIP cycle for a specific equipment type and/or a specific configuration of an industrial production line, which may lead to an increased prediction accuracy.

According to a 14^{th} aspect, a trained machine learning model for predicting the quality of a CIP cycle of an industrial production line comprising at least one equipment is provided, wherein the trained machine learning model is trained according to one of the 11^{th} to 13^{th} aspect.

The aforementioned advantages apply to this aspect.

According to a 15^{th} aspect, a computer program comprising instructions is provided, which when executed by a computer, causing the computer to perform the method of any one of 1^{st} to 8^{th} or 11^{th} to 13^{th} aspects.

The aforementioned advantages apply to this aspect.

### Brief description of the figures

Various aspects of the present invention are described in more detail in the following by reference to the accompanying figures without the present invention being limited to the aspects of these figures.
- Fig. 1: depicts a structure of an industrial production line including equipment and a CIP station.
- Fig. 2: depicts a CIP cycle including a plurality of CIP steps.
- Fig. 3: depicts a diagram of parameters produced during a CIP cycle of an industrial production line according to aspects of the present disclosure.
- Fig. 4: depicts a diagram of parameters produced during a CIP cycle of a separation device according to aspects of the present disclosure.
- Fig. 5: depicts a structure of an industrial production line including equipment, a CIP station, and a CIP analysis module according to aspects of the present disclosure.
- Fig. 6: depicts a CIP analysis module, a display device, and an equipment according to aspects of the present disclosure.
- Fig. 7: depicts a diagram showing temperature profiles of a plurality of CIP cycles according to aspects of the present disclosure.
- Fig. 8: depicts a process of data collection, preparation, model training and model deployment according to aspects of the present disclosure.

### Detailed description of preferred embodiments

In the following, the invention is described with reference to the accompanying figures in more detail. However, the present invention can also be used in other embodiments not explicitly disclosed hereafter. As detailed below, the embodiments are compatible with each other, and individual features of one embodiment may also be applied to another embodiment.

Throughout the figures and description, the same reference numerals refer to the same elements, unless stated otherwise. The figures may not be drawn to scale, and the relative size, proportions, and depiction of elements in the figures may be exaggerated for the purpose of clarity, illustration, and convenience. The figures do not limit the scope of the claims but merely support the understanding of the invention.

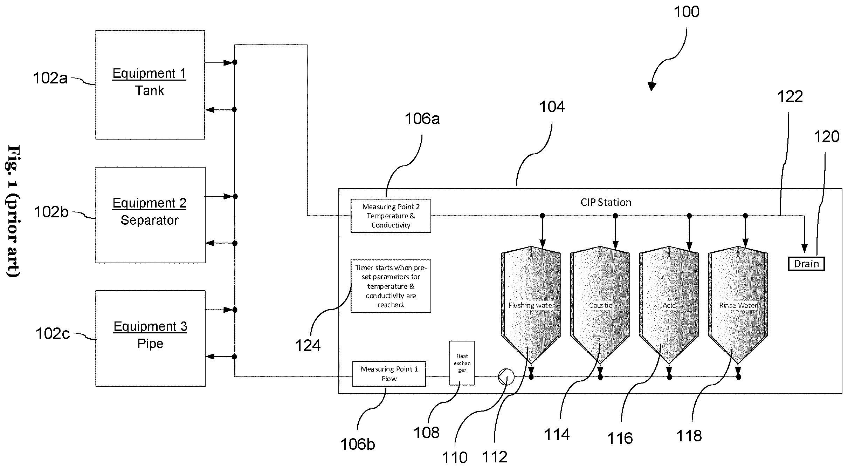

Fig. 1 illustrates a standard setup of a CIP station 104 integrated into an industrial production line 100, as for example used in the food and beverage or dairy industry.

The setup comprises the CIP station 104 integrated into the industrial production line 100. The industrial production line 100 comprises three system components: a tank 102a, a centrifugal separator 102b, and piping 102c. However, the industrial production line 100 may also comprise further or other components. Typical measuring points of a CIP Station are in the feed for the flow as indicated by measuring point 106b, and in the return flow of the liquid to the CIP Station 104, which may measure temperature and conductivity as indicated by measuring point 106a. The CIP station 104 further comprises a flushing water tank 112, a caustic tank 114, an acid tank 116 and a rinse water tank 118. All tanks 112, 114, 116 and 118 are connected to measuring point 106b, preferably over a regulating device 110 (e.g., a pump) and a heat exchanger 108. During a CIP cycle 200, the content of the tanks, also known as cleaning solution or CIP solution, is pumped through the heat exchanger 108 and the regulating device 110 into the equipment 102a-c and into the pipelines of the industrial production line 100 for cleaning purpose. The content is then passed back to the CIP station via pipeline 122 and may be forwarded to the drain 120. Hence, all the components are connected by pipes forming a loop line. A timer 124 controls, when, for example, the individual content of the tanks 112, 114, 116 and 118 is pumped into the loop that connects the equipment 102a-c to the CIP station.

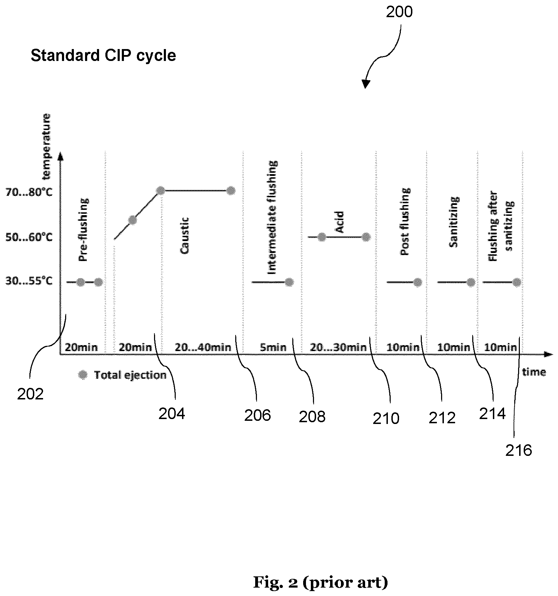

Fig. 2 illustrates a standard CIP cycle, also called CIP procedure or CIP sequence, that may be performed by CIP station 104 for a separator, like equipment 102b of industrial production line 100. The CIP Cycle 200 contains a sequence of different steps 202-216 and different cleaning agents applied during the different steps. In a pre-flushing step 202, which is typically the first step of a CIP cycle 200, the product in the pipes of the industrial production line 100 and, optionally, in the separator will be pushed out by water and major product residues are cleaned from the surface. If, for example, the industrial production line 100 is used for processing milk or milk products, in pre-flushing step 202, milk residue is pushed out of the industrial production line. The water used in this step typically has a temperature between 30-55 C°. The time required to perform step 202 is approximately 20 minutes.

In heating step 204, a caustic liquid is heated up, preferably up to 70-80 C°, which is typically done within 20 minutes. Then, in caustic step 206, the heated caustic liquid is pushed in the loop line. When the temperature and conductivity target is reached, a timer 124 may be started for a defined process time for the caustic step 206, which may vary between approximately 20-40 minutes. Caustic is highly alkaline and can effectively dissolve and remove organic soils, proteins, fats, and other residues commonly found in food processing equipment and pipelines. It can break down complex soils and facilitate their removal during the cleaning process.

In intermediate flushing step 208, the caustic liquid is pushed out of the loop line and of the equipment 102a-c with water. The water used in this step has preferably a temperature of 30-55 C° and takes approximately 5 minutes.

In acid step 210, an acid liquid is pushed in and run in the loop. The acid has preferably a temperature of 50-60 C°. Conducting step 210 requires approximately 20-30 minutes. In post-flushing step 212, the acid liquid is pushed out of the loop by water. The time required for this step is approximately 10 minutes, whereas the water has a temperature of preferably 30-55C°. Acidic solutions, such as nitric acid or phosphoric acid, are effective at dissolving and removing mineral deposits like calcium carbonate, calcium phosphate, and other scale formations that can accumulate on surfaces within processing equipment and pipelines. These deposits can hinder heat transfer efficiency, reduce flow rates, and harbor bacteria, so their removal is crucial for maintaining equipment performance and cleanliness.

Step 212 may be followed by sanitizing step 214. In which a disinfection liquid is pushed in and run in the loop. The disinfection liquid has a temperature preferably of 30-55 C° and the time required for carrying out this step is approximately 10 minutes. Sanitizing helps to eliminate or reduce microbial populations on surfaces, preventing microbial contamination of subsequent batches of product. This is particularly important in industries such as food and beverage processing, pharmaceuticals, and healthcare where maintaining hygienic conditions is critical to product safety and quality.

After step 214 a flushing-after-sanitization step 216 may be performed. During this step, the disinfection liquid is pushed out of the loop, preferably by water with a temperature of preferably 30-55C°. The time for performing this step is preferably 10 minutes.

Depending on the product processed in the industrial production line 100 and/or depending on the specific setup of the industrial production line 100, different variations of the CIP cycle 200 may be carried out. This is because one product may require a longer and more intensive cleaning, whereas another product may require shorter cleaning sequences. Individual steps of CIP cycle 200 may also be skipped or circumvented. Hence, the cleaning procedure conducted by CIP cycle 200 is a dynamic process which may vary and change.

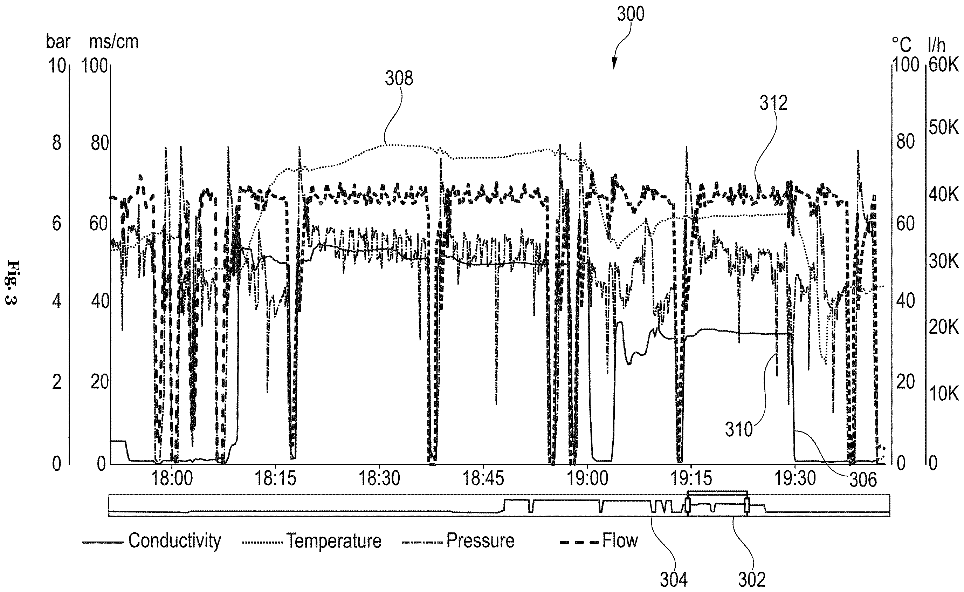

Fig. 3 illustrates a diagram 300 of parameters produced during a CIP cycle 200 of an industrial production line 100. The curves 306, 308, 310 and 312 indicate the behavior of the corresponding parameters recorded during slot 302 of the complete parameter recording 304. Hence, the diagram 300 depicts only a time extract of a complete parameter measurement.

Curve 306 indicates the progression of the conductivity in slot 302, which may for example be measured at measurement point 106a of CIP station 104. Conductivity measurement is used to monitor the concentration of cleaning agents and the level of contamination in the cleaning solution. During the CIP cycle 200, conductivity should decrease as contaminants are removed from the equipment and diluted in the cleaning solution. A significant decrease in conductivity indicates effective cleaning. Conversely, a constant or increasing conductivity level suggests incomplete cleaning or insufficient dilution of cleaning agents, which can compromise product quality and equipment hygiene.

Curve 308 indicates the progression of the temperature in slot 302, which may for example be measured at measurement point 106a of CIP station 104. Temperature monitoring ensures that the cleaning solution reaches the desired temperature and maintains it throughout the CIP cycle 200. Deviations from the specified temperature range can indicate issues such as inadequate heating equipment, insufficient contact time, or improper mixing of cleaning chemicals.

Curve 310 indicates the progression of the pressure in slot 302, which may for example be measured at measurement point 106b of CIP station 104. Pressure monitoring helps ensure proper distribution and circulation of the cleaning solution throughout the equipment. Adequate pressure is necessary to dislodge and remove contaminants from surfaces effectively. Insufficient pressure may result in areas with poor cleaning coverage, leading to microbial growth or product contamination. Monitoring pressure variations during the CIP cycle 200can identify potential blockages, leaks, or equipment malfunctions that could impede the cleaning process and compromise the quality of the final product.

Curve 312 indicates the progression of the flow in slot 302, which may for example be measured at measurement point 106b of CIP station 104. Flow rate measurement is essential for assessing the efficiency of cleaning solution delivery and drainage during the CIP cycle 200. Consistent flow rates indicate uniform distribution of the cleaning solution, ensuring thorough cleaning of all equipment surfaces. Fluctuations or interruptions in flow may indicate blockages, valve or regulating device malfunctions, or insufficient pump capacity, which can hinder the cleaning process and compromise product quality.

The values of the aforementioned parameters of a typical CIP cycle 200 are thus not constant and will change during the individual steps 202, 204, 206, 208, 210, 212 and 214.

To evaluate the cleaning quality of a CIP cycle 200 on individual equipment level, e.g. for one of equipment 102a.c, it is necessary to perform an equipment-dependent analysis of the cleaning steps 202, 204, 206, 208, 210, 212 and 214 required for the specific equipment 102a-c. Examples of equipment specific cleaning steps for a centrifugal separator are ejections, overflow, spray ball, bowl bottom flush, and discharge pressure.

Ejections: The ejection during a cleaning process cleans the disks of the product deposits. Also, the CIP Liquid will be cleaned.

Overflow: With the overflow, the centripetal pump chamber of the separator is overflowing which leads to a cleaning of the complete centripetal pump chamber and the outside of the separator bowl. In this step, the discharge pressure reaches its limit for a few seconds.

Spray ball: The spray ball is a cleaning device in the solid vessel. It will be active for a short period of time between the ejections.

Bowl bottom flush: The spray nozzle will be active for a short period of time between the ejections.

Discharge pressure: for a proper covering of the centripetal pumps, the discharge pressure of the discharge liquids is controlled to secure a proper cleaning.

To properly analyze the cleaning quality of the separator, specific sensor readings from the separator are recorded and analyzed. Examples of parameters used for CIP quality analysis of a separator may consist of motor current, bowl speed, total ejection/partial ejection, and vibration.

Motor current is an indicator of the power consumption and load on the separator's motor during operation. Abnormal increases in motor current during the CIP cycle 200 may indicate issues such as excessive friction, improper alignment, or the presence of solid residues causing increased resistance. Monitoring motor current can help detect potential mechanical problems or insufficient cleaning, ensuring that the separator operates efficiently and reliably after the CIP cycle 200.

Bowl speed refers to the rotational speed of the centrifugal bowl in the separator. Maintaining the correct bowl speed during the CIP cycle 200 is essential for effective separation of solids and liquids and efficient cleaning. Deviations from the specified bowl speed can affect separation efficiency and cleaning effectiveness. Monitoring bowl speed ensures that the separator operates within the optimal range, facilitating thorough cleaning and preventing damage to the equipment.

Ejection refers to the discharge of separated solids or liquids from the separator. Total ejection involves complete removal of accumulated solids or separated phases from the separator, while partial ejection may leave behind residual contaminants. Monitoring ejection characteristics during the CIP cycle 200 ensures that the separator effectively removes all contaminants and residues, preventing cross-contamination and maintaining product quality. Inadequate ejection or incomplete removal of solids may indicate insufficient cleaning or operational issues, requiring adjustments to the CIP process parameters.

Vibration monitoring helps detect mechanical problems, such as misalignment, imbalance, or bearing wear, which can affect the separator's performance and reliability. Excessive vibration during the CIP cycle 200 may indicate issues with the rotating components or the overall structural integrity of the separator. Monitoring vibration levels helps identify potential maintenance needs or operational issues that could compromise the quality of the CIP process or the performance of the separator.

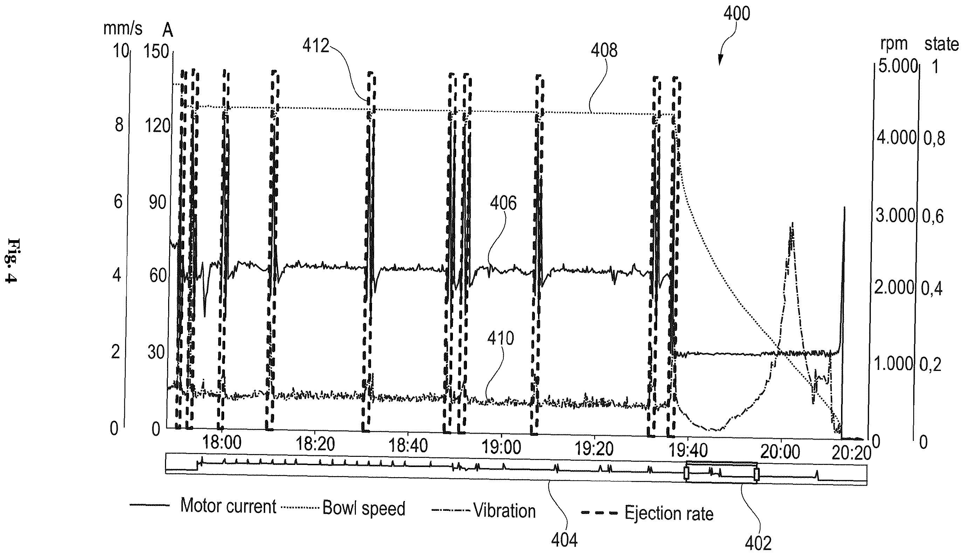

Fig. 4 illustrates a diagram 400 of parameters produced during a CIP cycle of a separation device (e.g., a separator or decanter). The depicted curves, 406, 408, 410 and 412 indicate parameter values recorded in slot 402 of the complete parameter recording 404. Curve 406 indicates the progression of the motor current in slot 402, which may for example be measured with a sensor arranged in a decanter or separator, like equipment 102b of industrial production line 100. Curve 408 indicates the progression of the bowl speed in slot 402, which may for example be measured with a sensor arranged in a decanter or separator, like equipment 102b of industrial production line 100. Curve 410 indicates the progression of the vibration in slot 402, which may for example be measured with a sensor arranged in a decanter or separator, like equipment 102b of industrial production line 100. Curve 412 indicates the progression of the ejection rate in slot 402, which may for example be measured with a sensor arranged in a decanter or separator, like equipment 102b of industrial production line 100.

As can be seen from Fig. 4, the curves 406, 408 and 410, indicating the motor current, the bowl speed and the vibration, respectively, are almost constant and only significantly change or vary during an ejection.

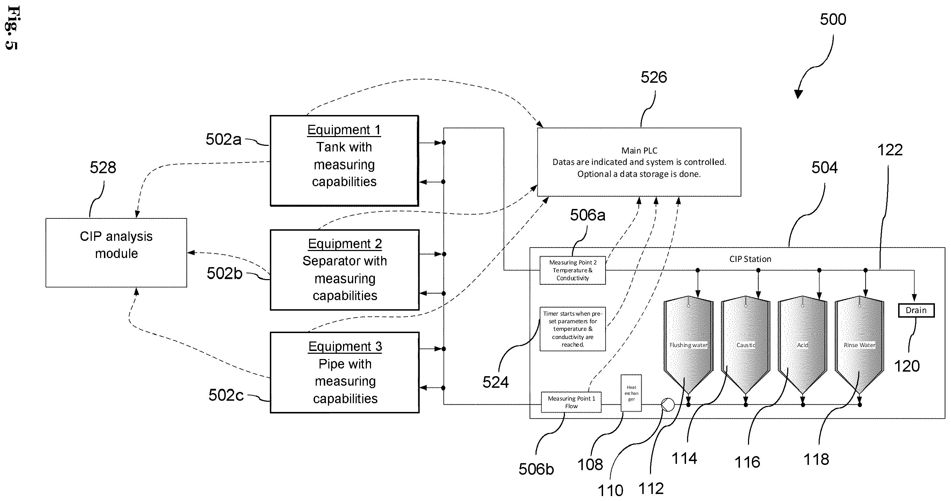

Fig. 5 illustrates a structure of an industrial production line 500 including equipment 502a-c, a CIP station 504 including measurement points 506a-b, a regulating device 110, a heat exchanger 108, tanks 112, 114, 116, 118 and a CIP analysis module 528 as well as a main programmable logic controller (PLC) 526 according to aspects of the present disclosure. Compared to industrial production line 100, the equipment 502a-c comprise measurement capabilities, e.g. in terms of sensors, that allow to measure equipment specific parameters, like temperature, conductivity, and flow. If the equipment 502a-c is a decanter or separator, the equipment specific parameters may additionally or alternatively comprise bowl speed, motor current, ejection rate and vibration. It is apparent for the skilled reader that, dependent on the equipment type, further parameters may be measured. Like measurement points 106a-b, measurement points 506a-b may measure parameters of the CIP station 504, like temperature, conductivity, pressure, and flow. The functionality of timer 524 generally corresponds to the functionality of timer 124. The additional equipment specific measurements are collected and stored together with the standard CIP measurements from the CIP cycle 200.

Across production lines in the food and beverage industry multiple different configurations of CIP cycles 200 may be chosen, depending on the degree of fouling of the units of the industrial production line 500, cleaning need and the runtime of the specific equipment 502a-c, e.g. a separator, heat exchanger or evaporation plant. Consequently, pre-defined CIP cycles can be chosen by the main PLC 526, which differ mainly in the number of cleaning solutions used and the time the total CIP cycle 200 is running. Furthermore, the length of the individual steps 202-216 may vary between different configurations of a CIP cycle 200.

To determine the quality, e.g. the cleaning effectiveness, of a CIP cycle 200, a detailed analysis and evaluation of a CIP cycle 200 is required. Every cleaning step 202-216 may be required to be analyzed individually. Additionally, it may be advantageous to record and analyze general CIP cycle characteristics provided from the CIP Sation 500 in combination with equipment specific measurements.

Therefore, one or more processors may be operably connected to both the equipment 502a-c and the CIP station 504 and configured to run a software configured to obtain sensor data and perform the necessary data analysis. This may be done by CIP analysis module 600, preferably in conjunction with main PLC 526.

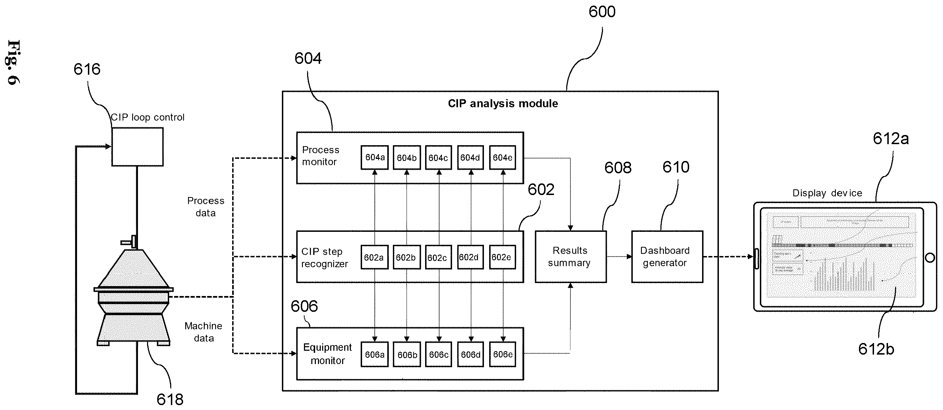

Fig. 6 illustrates a detailed view of CIP analysis module 600, a display device 612a displaying a dashboard 612b, and an equipment 618 connected to a CIP loop control 616 according to aspects of the present disclosure. The CIP analysis module 600 is preferably realized in software, hardware, or a combination thereof. CIP analysis module 600 may comprise three main components: a CIP step recognizer 602, a process monitor 604, and an equipment monitor 606.

The CIP step recognizer 602 identifies the current step 202-216 of a CIP cycle 200 by means of CIP step identifiers 602a-e and orchestrates the execution of suitable machine learning models 604a-e, 606a-e. The identification of the current CIP step 202-216 may be done by the CIP step identifiers 602a-e via data exchange with the main PLC 526. Hence, the main PLC 526 may simply communicate to the CIP analysis module 526 which CIP step 202-216 is presently active. Alternatively, the current CIP step 202-216 may be determined by the CIP step identifiers 602a-e of CIP step recognizer 602 using sensor values indicating the temperature and conductivity, which may for example be provided by a measuring point 106a, 506a of the used CIP station 104, 504. This is possible since each CIP step 202-216 typically operates with a different temperature and conductivity range. Based on these values, the selection of the desired model(s) could be steered, too.

Alternatively, the CIP step identifiers 602a-e of CIP step recognizer 602 may identify a plurality of pre-configured CIP cycle variations. In this case, a CIP step 202-216 may be understood as complete or partial CIP cycle 200. This process may be performed as a batch analysis process, as the entire CIP cycle 200 needs to be finished before the analysis can be performed.

The process monitor 604 may comprise a plurality of trained process-specific machine learning model 604a-e, at least one for each different type of CIP step 202-216. The trained process-specific machine learning model 604a-e are trained for the overall process data and are not equipment specific. The execution of the trained process-specific machine learning models 604a-e is triggered from the CIP step recognizer 602. Hence, the CIP step recognizer 602 selects an appropriate trained process-specific machine learning model 604a-e for each identified CIP step 202-216.

Process data, e.g. obtained from measuring points 106a-b, 506a-b and/or process data obtained from the measurement capabilities of equipment 502a-c is received by each of the selected trained process-specific machine learning models 604a-e. Based on this information, the trained process-specific machine learning models 604a-e perform a prediction to determine the cleaning quality of the individual CIP steps 202-216 and thus also of the complete CIP cycle 200.

Equipment monitor 606 comprises a plurality of trained equipment-specific machine learning models 606a-e, at least one per CIP step 202-216. The different trained equipment-specific machine learning models 606a-e are specialized for the anomaly detection of the equipment process. A trained equipment-specific machine learning model 606a-e may, for example, have been trained to be used in conjunction with separators, whereas other trained equipment-specific machine learning models 606a-e may have been trained for being used with tanks or other device types. The selection and execution of the trained equipment-specific machine learning models 606a-e is triggered by the CIP step recognizer 602. Process data, e.g. obtained from measuring points 106a-b, 506a-b and/or process data obtained from the measurement capabilities of equipment 502a-c is received by each of the selected trained equipment-specific machine learning models 606a-e. Based on this information, the trained equipment-specific machine learning models 606a-e perform a prediction to determine the cleaning quality of the individual CIP steps 202-216 and thus also of the complete CIP cycle 200.

The plurality of trained process and equipment specific machine learning models 604a-e, 606a-e in both the process monitor 604 and the equipment monitor 606 are reflecting either a complete or a partial CIP cycle 200 identified by the CIP step recognizer 602. A plurality of different CIP cycle configurations may be constructed and the trained models 604a-e, 606a-e may have been trained for at least one of these configurations. The following listing depicts three models that have been trained for a standard CIP cycle 200, a short CIP cycle and a special CIP cycle that differ in the conducted CIP steps:
- Model 1 (Standard) = Water - Caustic - Water - Acid - Water
- Model 2 (Short) = Water - Caustic - Water
- Model 3 (Special) = Water - Acid - Water - Caustic - Water

Alternatively, the CIP step recognizer 602 is configured to treat the individual steps 202-216 in the CIP cycle 200 individually. Sensor input data may be prepared by the CIP step recognizer 602 according to each step 202-216 and tagged to define the single steps, like flushing and caustic. In this case, each of the trained process and equipment specific machine learning models 604a-e, 606a-e relate to one specific CIP step 202-216 (and not to a complete CIP cycle 200 as outlined above) and have been trained accordingly.

CIP step recognizer 602 may automatically choose the best fitting model for a CIP step 202-216 using its CIP step identifiers 602a-e. Hence, the CIP analysis module 600 may be adapted to any variation of the applied CIP cycle 200 in a specific production context.

Accordingly, the CIP step recognizer 602 identifies via its CIP step identifiers 602a-e a CIP step 202-216 or a sequence of CIP steps 202-216 of a CIP cycle 200 and communicates this information to both the process monitor 604 and to equipment monitor 606. Based on the information, the process monitor 604 selects a properly trained process-specific machine learning model 604a-e, one per CIP step 202-216 or one per sequence of CIP steps 202-216.

The same information is also communicated to the equipment monitor 606. Based on the information, the equipment monitor 604 selects an properly trained equipment-specific machine learning model 606a-e, one per CIP step 202-216 or one per sequence of CIP steps 202-216.

During operation, the process monitor 604 and the equipment monitor 606 obtain operation data of the CIP station 504 and from equipment 502a-c, 618 of the industrial production line 500. The operation data may refer to an individual CIP step 202-216 or to a sequence of CIP steps 202-216. For each CIP step 202-216 or for each sequence of CIP steps 202-216, the operation data is passed to the corresponding trained model 604a-e, 606a-e of the process monitor 604 and of the equipment monitor 606. The operation data may comprise parameters as discussed with respect to Figs. 3 and 4. Based on the operation data, each of the models 604a-e, 606a-e performs a prediction to indicate the execution quality of the corresponding CIP step 202-216 of the sequence of CIP steps 22-216. The quality indicates how well each step 202-216 or sequence was executed.

The prediction results of all machine learning models 604a-e, 606a-e are summarized through different methods like the ensemble learning method bagging, which may be performed by results summary module 608. The results may be forwarded to dashboard generator module 610 to be visualized in a dashboard 612b displayed on a display device 612a for the user.

To predict the quality, an anomaly detection analysis may be performed by the plurality of the trained machine learning models 604a-e, 606a-e of the process monitor 604 and of the equipment monitor 606. As an input, as outlined above with respect to Figs. 3 and 4, the trained process-specific machine learning models 604a-e may obtain the sensor data of conductivity, temperature, discharge pressure and flow. These parameters allow to recognize anomalies in a CIP step 202-216 or in a sequence of CIP steps 202-216. Likewise, the trained equipment-specific machine learning models 606a-e of the equipment monitor 606 may obtain parameters like bowl speed, motor current, vibration and total ejection of a corresponding equipment 502a-c, 618. These parameters allow to recognize anomalies in a CIP step 202-216 or in a sequence of CIP steps 202-216.

All the aforementioned parameters may be provided by corresponding sensors of the CIP station 500 and/or of the equipment 502a-c, 618 with a minimal sampling rate of one second. The parameters may be buffered during the duration of the CIP procedure, such that a complete data set is provided the CIP analysis module 600 as a batch data analysis process. Alternatively, the data may be provided to the CIP analysis module 600 in semi real-time when the CIP cycle 200 may change state or phase. Then, the data associated with the previous phase is passed to the CIP analysis module 600.

The parameters of each CIP cycle 200 may be stored in a sequence named with a unique identification (ID) number. The trained machine learning model may generate an aggregate quality score at the end of a complete CIP cycle 200. The quality may be expressed in terms of an anomaly score. Examples of aggregated anomaly scores and the corresponding cleaning quality interpretation may look like as follows:
- 0-20%: CIP cycle 200 was executed in good condition.
- 21-70%: Performance of CIP cycle 200 has to be checked.
- 71-100%: CIP cycle 200 has to be repeated.

Suitable examples of algorithms that may act as the basis for the machine learning models 604a-e, 606a-e are Support Vector Machines, Optimized Support Vector Machines, Random Forests, Optimized Random Forests, Nearest Neighbors, Optimized Nearest Neighbors, Autoencoders or Self Organizing Maps (SOMs).

A support vector machine (SVM) is a supervised learning algorithm used for classification and regression tasks. It finds the optimal hyperplane that best separates different classes in the feature space, maximizing the margin between the classes. An optimized support vector machine refers to enhancements made to traditional SVMs, such as kernel tricks, parameter tuning, and algorithmic optimizations, to improve performance in terms of accuracy, training time, and generalization to unseen data. These optimizations help SVMs handle more complex data and achieve better results in real-world applications.

Random forests are an ensemble learning method used for classification and regression tasks. They consist of a collection of decision trees trained on random subsets of the data and features. During prediction, the output of multiple trees is combined to produce a final prediction, offering robustness against overfitting and high accuracy.

Optimized random forests refer to improvements made to traditional random forests, such as tuning hyperparameters, selecting optimal feature subsets, and employing advanced tree-building techniques like bootstrapping and feature bagging. These optimizations enhance the performance, scalability, and interpretability of random forests, making them more effective in handling complex datasets and achieving better predictive accuracy.

A nearest neighbor algorithm classifies new data points based on the similarity to existing labeled data points. It assigns the label of the nearest data point(s) to the new point, making it a simple and intuitive classification method.

An optimized nearest neighbor algorithm involves strategies such as using efficient data structures like KD-trees or ball trees to speed up the search for nearest neighbors, optimizing distance metrics, and applying dimensionality reduction techniques to handle high-dimensional data. These optimizations aim to reduce computational complexity and improve the algorithm's scalability and performance, making it more suitable for large datasets and real-world applications.

An autoencoder is a type of neural network architecture used for unsupervised learning, particularly in data compression and feature learning. It consists of an encoder network that compresses the input data into a lower-dimensional representation called a latent space, and a decoder network that reconstructs the original input data from this compressed representation. By minimizing the reconstruction error, autoencoders learn to capture the most important features of the input data, enabling tasks such as dimensionality reduction, data denoising, and anomaly detection.

A SOM is a special type of neural network. A SOM consists of several neurons positioned on a grid. Each neuron contains a weight vector with the same length as the input samples and randomly initialized values. During training, each input sample is applied by calculating the nearest neuron and "pulling" the neuron in the direction of the sample.

To use SOMs for CIP cycle analysis, the different sensors of the CIP station 500 and/or of the equipment 502a-c, 618 need to be processed separately in one SOM. This approach ensures that the root cause for an anomaly can be localized regarding both the responsible sensor and a specific point in time. To train a model, the CIP cycles 200 provided for training are set to a standardized machine-depending length. This is to ensure the ability of the SOM algorithm to interpret one CIP cycle 200 as one datapoint while transferring the time axis to the SOM's dimensions. Doing so removes the absolute time axis from the analysis and enables the SOM to compare the data based on the time since the cycle started.

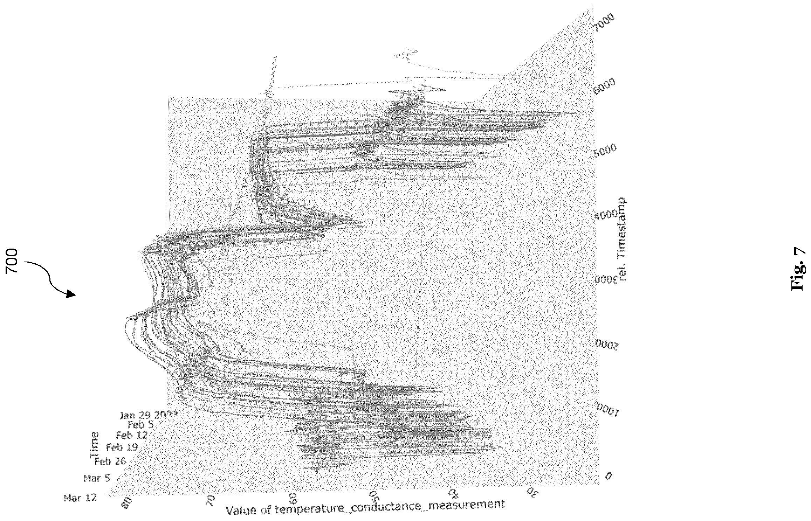

Fig. 7 shows a diagram 700 that visualizes the SOM-based approach by means of temperature conductance profiles that have been monitored over the course of several CIP cycles 200 by choosing a perspective along the absolute time axis and perpendicular to the value and relative timestamp axes. The x-axis of diagram 700 represents the relative timestamps, the y-axis of diagram 700 represents the value of the temperature conductance, and the z-axis of diagram 700 represents dates when the individual measurements have been conducted.

Choosing a SOM as machine learning algorithm has several advantages compared to the usage of other types of machine learning algorithms. First, SOM models output a high degree of details by assigning each input vector a same length vector of anomaly scores. This characteristic also enables further high-level aggregations while providing the base for fine-grained root cause analysis. One main purpose of aggregated anomaly scores is the evaluation of the quality of a certain CIP cycle 200. By aggregation the scores on a weekly basis, long-term trend analysis can be done. For aggregation over multiple consecutive CIP cycles 200, the median of the CIP's anomaly scores is used instead of the arithmetic mean in order to mitigate the effects of few outlier CIP cycles 200.

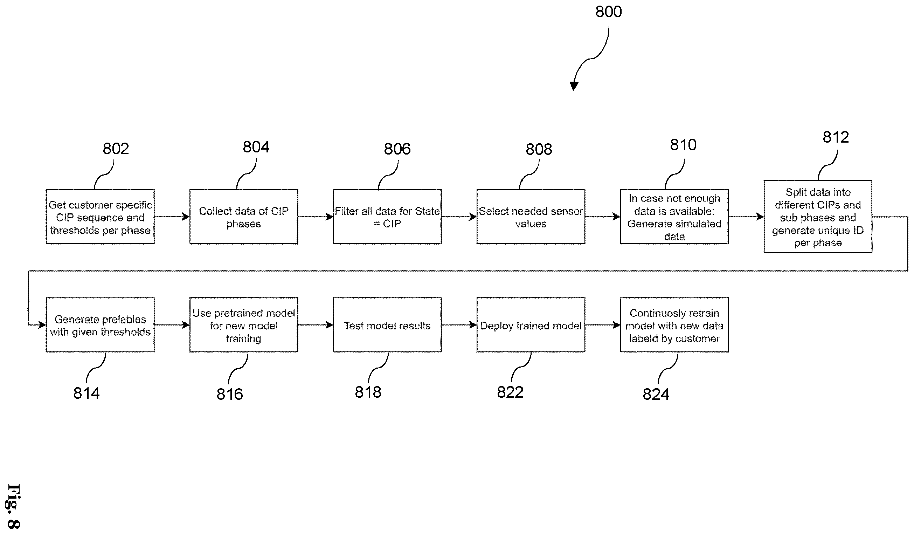

Fig. 8 illustrates a process 800 that may be used for training and deploying models. In step 802, specific configurations for CIP cycles 200 are obtained, wherein each step 202-216 of the CIP cycle 200 may have assigned specific sensor threshold values. In step 804, the configurations of the CIP cycles 200 are executed in a real industrial production line 500 that is connected to a CIP station 504. Sensor data is measured and recorded during this step to be used as training data. The training data may include following sensor values that may preferably be used for training process specific machine learning models: machine state, flow, pressure heavy phase, temperature, and conductivity. Additional sensor data for the training of equipment-specific machine learning models may be used and may comprise: bowl speed, motor current, ejection, ejection rate, and vibration.

In step 806, the obtained sensor data is filters such that sets of sensor data can be assigned to each CIP cycle 200. In step 808, the sensor data that is relevant may be selected. For example, data of sensors that are not relevant for the CIP analysis may be omitted. In step 810, which is an optional step, additional sensor data may be generated, e.g., by simulating the operation of an industrial production line 500 by way of computer simulation.

In step 812, the obtained sensor data is split to assign sensor data to the corresponding CIP cycle 200 or to the steps 202-216 of the corresponding CIP cycle 200. The sensor data may be formatted with a timestamp as timeseries data. The different time series values may be grouped in CIP batches with a unique identifier. To ensure a reliable model training, data from more than 75 CIP cycles 200 may be needed where at least 10 CIP batches can be classified as "good". The data obtained in this step is the actual training data.

In step 814, the training data may be prelabeled to reduce the effort of the model training. The pre-labels may be created with a set of threshold values defined by the customer for each CIP phase (see step 802), e.g.:
- Duration caustic phase (min/max + standard deviation)
- Temperature caustic phase (min/max + standard deviation)
- Conductivity caustic phase (min/max + standard deviation)

During the process of the training data preparation each CIP phase is checked for a threshold violation and labeled as "bad"/anomalous in case of a threshold violation.

In step 816, the actual training is performed. With these prelabeled training data, a data scientist or process expert may train a model from scratch or may further train an already pre-trained model. The specific trained models for a customer will be continuously retrained to improve their prediction quality.

In step 818, the prediction quality of the trained models may be tested, e.g., by operating the model in a simulated environment.

In step 822, the tested models may be deployed to be used in an CIP analysis module 600.

In step 824, the trained and deployed models may be continuously retrained during operation. During each CIP cycle 200, the sensor values are measured and saved. A user is asked to mark the finished CIP cycle 200 as "good" or "bad", e.g. on a human machine interface, like the display 612a. The labeled data is used for incremental retraining of the model. With this continuous retraining, more reliable prediction results can be achieved, for example if changes have been applied to the conducted CIP cycle. As an example, in case of semi real-time analyses, the system may be capable of detecting if machine-related functions have been missing during a CIP step 202-216. With this information, an equipment, e.g. a separator, can activate automatically an overflow or total discharge before allowing the system to jump into the next CIP step 202-216. This means, that a quality check of the single equipment CIP cycle 200 per CIP step 202-216 is done.

As outlined above, the created and labeled training data is used to train supervised anomaly detection machine learning models. All in all, there is a set of trained models, all specialized for another CIP step 202-216 or for a sequence of CIP steps 202-216. Per CIP step 202-216 or per sequence of CIP steps 202-216, at least two models are created, one trained process-specific machine learning model 604a-e and one trained equipment specific machine learning model 606a-e, both trained for anomaly detection. The created models are tested against a labeled test dataset and after having achieved satisfying results, the trained models 604a-e, 606a-e are transferred into the production system to continuously detect anomalies.

Embodiments of the present disclosure may be realized in any of various forms, e.g., in software. For example, in some embodiments, the present invention may be realized as a computer-implemented method, a computer-readable memory medium, or a computer system.

In some embodiments, a non-transitory computer-readable memory medium may be configured so that it stores program instructions and/or data, where the program instructions, if executed by a computer system, cause the computer system to perform a method, e.g., any of the method embodiments described herein, or, any combination of the method embodiments described herein, or, any subset of any of the method embodiments described herein, or, any combination of such subsets.

In some embodiments, a computing device may be configured to include a processor (or a set of processors) and a memory medium, where the memory medium stores program instructions, where the processor is configured to read and execute the program instructions from the memory medium, where the program instructions are executable to implement any of the various method embodiments described herein (or, any combination of the method embodiments described herein, or, any subset of any of the method embodiments described herein, or, any combination of such subsets). The device may be realized in any of various forms.

Although specific embodiments have been described above, these embodiments are not intended to limit the scope of the present disclosure, even where only a single embodiment is described with respect to a particular feature. Examples of features provided in the disclosure are intended to be illustrative rather than restrictive unless stated otherwise. The above description is intended to cover such alternatives, modifications, and equivalents as would be apparent to a person skilled in the art having the benefit of this disclosure.

The scope of the present disclosure includes any feature or combination of features disclosed herein (either explicitly or implicitly), or any generalization thereof, whether or not it mitigates any or all of the problems addressed herein. In particular, with reference to the appended claims, features from dependent claims may be combined with those of the independent claims and features from respective independent claims may be combined in any appropriate manner and not merely in the specific combinations enumerated in the appended claims.

## Claims

1. A computer-implemented method for predicting the quality of a Cleaning-in-Place, CIP, cycle (200) of an industrial production line (100) comprising at least one equipment (502a-c; 618) and at least one CIP station (104; 504), the method comprising the steps of:
executing, by the CIP station (104; 504), the CIP cycle (200);
identifying one or more CIP steps (202-216) of the CIP cycle (200);
selecting, for each of the identified one or more CIP steps (202-216), a trained process-specific machine learning model (604a-e) associated with the CIP station (104; 504) according to the identified one or more CIP steps (202-216);
passing one or more CIP station-specific parameters to the trained process-specific machine learning model (604a-e); and
predicting the quality of the CIP cycle (200) based on the one or more CIP station-specific parameters, wherein the quality is reflected by an output of each of the trained process-specific machine learning models (604a-e).

2. The method of claim 1, further comprising the steps of:
selecting, for each of the identified one or more CIP steps (202-216), a trained equipment-specific machine learning model (606a-e) associated with the at least one equipment (502a-c; 618) of the industrial production line (100);
passing one or more equipment-specific parameters to the trained equipment-specific machine learning model (606a-e); and
wherein the predicting the quality of the CIP cycle (200) is further based on the one or more equipment-specific parameters, wherein the quality is reflected by an output of each of the trained equipment-specific machine learning models (606a-e).

3. The method of claim 2, wherein the predicting the quality of the CIP cycle (200) further comprises:
aggregating, when the CIP cycle (200) is completed, the outputs of each of the trained process-specific machine learning models (604a-e) and of each of the trained equipment-specific machine learning models (606a-e) to generate a quality indicator.

4. The method of claims 2-3, wherein the at least one equipment (502a-c; 618) is a separation device, preferably a decanter or a separator; and
wherein the one or more equipment-specific parameters comprise one or more of:
- a bowl speed of the at least one equipment (502a-c; 618);
- a motor current of the at least one equipment (502a-c; 618);
- an ejection indicator indicating whether a total ejection or a partial ejection has been conducted by the at least one equipment (502a-c; 618);
- a vibration rate of the at least one equipment (502a-c; 618).
- a flow rate to the equipment; and/or
- a discharge pressure or backpressure rate.

5. The method of any one of the preceding claims, wherein the one or more CIP station-specific parameters comprise one or more of:
- a state of the industrial production line (100);
- a flow rate;
- a backflow temperature;
- a line temperature and/or
- a conductivity.

6. The method of claim 5, wherein the step of identifying one or more CIP steps (202-216) of the CIP cycle (200) is based on at least one of:
- the backflow temperature and/or the conductivity;
- data provided by the CIP station and/or by the production line.

7. The method of any one of the preceding claims, wherein the trained process-specific machine learning model (604a-e) is one of:
- a Support Vector Machine, preferably an Optimized Support Vector Machine;
- a Random Forest, preferably an Optimized Random Forest;
- a Nearest Neighbors algorithm, preferably an Optimized Nearest Neighbors algorithm,
- an Autoencoder;
- one or more Self Organizing Maps, SOMs.

8. The method of any one of the preceding claims 2-4, wherein the trained equipment-specific machine learning model (606a-e) is one of:
- a Support Vector Machine, preferably an Optimized Support Vector Machine;
- a Random Forest, preferably an Optimized Random Forest
- a Nearest Neighbors algorithm, preferably an Optimized Nearest Neighbors algorithm,
- an Autoencoder;
- one or more Self Organizing Maps, SOMs.

9. A control computing system for predicting the quality of a Cleaning-in-Place, CIP, cycle (200) of an industrial production line (100) comprising at least one equipment (502a-c; 618), the control computing system comprising means for performing the method of any one of the claims 1-8.

10. An industrial production line (100) comprising or communicatively coupled to at least one control computing system according to claim 9.

11. A computer-implemented method for training a process-specific machine learning model adapted to predict the quality of a CIP cycle (200) of an industrial production line (100), the method comprising:
obtaining training data from a plurality of parameter sets comprising at least one of:
- a state of the industrial production line;
- a flow rate;
- a discharge pressure rate;
- a backflow temperature; and/or
- a conductivity; and
training the process-specific machine learning model based on the training data to generate a trained process-specific machine learning model (604a-e).

12. A computer-implemented method for training an equipment-specific machine learning model adapted to predict the quality of a CIP cycle (200) of an industrial production line (100), wherein the equipment-specific machine learning model is associated with a separation device, preferably a decanter or a separator, the method comprising:
obtaining training data from a plurality of parameter sets comprising at least one of:
- a bowl speed of the separation device;
- a motor current of the separation device;
- an ejection indicator indicating whether a total ejection or a partial ejection has been conducted by the separation device; and/or
- a vibration rate of the separation device; and
training the equipment-specific machine learning model based on the training data to generate a trained equipment-specific machine learning model (606a-e).

13. The method of any one of the claims 11-12, wherein the method further comprises:
obtaining further training data from an industrial production line during operation;
retraining the trained process-specific machine learning model (604a-e) or the trained equipment-specific machine learning model (606a-e) based on the further training data.

14. A trained machine learning model for predicting the quality of a Cleaning-in-Place, CIP, cycle (200) of an industrial production line (100) comprising at least one equipment (502a-c; 618), wherein the trained machine learning model is trained according to the method of any one of the claims 11-13.

15. A computer program comprising instructions, which when executed by a computer, causing the computer to perform the method of any one of claims 1-8 or 11-13.
